# FASCICULE DE BREVET EUROPEEN

(11) **EP 1 619 962 B1**
(45) Date de publication et mention de la délivrance du brevet: **06.06.2007**
(21) Numéro de dépôt: 04702734.7
(22) Date de dépôt: 16.01.2004
(51) Int. Cl.: A23L 1/00, A23L 1/30

(54) **COMPOSITION POUR UNE ADMINISTRATION PAR VOIE ORALE CONTENANT DES CAPSAICINOIDES**
CAPSAICINOID ENTHALTENDE PHARMAZEUTISCHE ZUSAMMENSETZUNG ZUR ORALEN VERABREICHUNG
COMPOSITION FOR ORAL ADMINISTRATION CONTAINING CAPSAICINOIDS

(30) Priorité: 17.01.2003 FR 0300506
(43) Date de publication de la demande: 01.02.2006
(73) Titulaire: Institut Phytoceutic, 83600 Frejus (FR)
(72) Inventeur: VERNEAU, Bernadette, F-83600 Frejus (FR)
(74) Mandataire: Ahner, Francis
(86) Numéro de dépôt international: PCT/FR2004/000085
(87) Numéro de publication internationale: WO 2004/064542

(56) Documents cités:
- EP-A- 0 201 041
- WO-A-00/41708
- WO-A-97/33599
- WO-A-98/47376
- FR-A- 2 776 189
- GB-A- 1 201 650
- US-A- 3 619 212
- US-A- 5 273 754
- US-A- 6 022 718
- US-A1- 2002 028 257
- DATABASE WPI Section Ch, Week 200165 Derwent Publications Ltd., London, GB; Class D13, AN 2001-571255 XP002256558 & CN 1 304 682 A (DONG S) 25 juillet 2001 (2001-07-25)
- DATABASE WPI Section Ch, Week 200028 Derwent Publications Ltd., London, GB; Class B04, AN 2000-321173 XP002285380 & JP 2000 072642 A (LION CORP) 7 mars 2000 (2000-03-07)

## Description

La présente invention a pour objet une composition pour une administration par voie orale contenant des capsaicinoïdes et son utilisation comme médicament, aliment, complément alimentaire ou produit diététique. Cette composition présente l'avantage d'être dépourvue d'effet irritant sur la muqueuse gastrique.

Le fruit du Piment, frais ou sec, est généralement utilisé comme épice. Son pouvoir piquant au niveau de la cavité buccale est fonction de sa teneur en capsaicinoïdes.

Les capsaicinoïdes sont constitués majoritairement de capsaïcine, et minoritairement d'homocapsaïcine et de dihydrocapsaïcine.

Des travaux scientifiques réalisés depuis plusieurs années ont permis de démontrer que les capsaicinoïdes contenus dans le Piment (ou capsicum) ont la capacité de stimuler la thermogenèse.

La thermogenèse est le développement continu et régulier de la chaleur chez les êtres vivants. Une des méthodes pharmacologiques reconnue actuellement pour le traitement et la prévention de l'obésité est la stimulation de la thermogenèse, entraînant une augmentation de la dépense énergétique et donc une consommation plus importante de calories.

L'augmentation de la thermogenèse par les capsaicinoïdes contenus dans le Piment est liée à une stimulation du système sympathique. L'aspect métabolique de cette augmentation de la thermogenèse est différent selon les études. Dans certaines études, elle est liée à une augmentation de l'oxydation des lipides. Dans d'autres études, elle est liée à l'augmentation de l'oxydation des glucides. Néanmoins, lorsque le repas est riche en lipides, l'oxydation de ces dernières devient prépondérante.

La capacité des capsaicinoïdes contenus dans le Piment à stimuler la - thermogenèse et favoriser l'oxydation des lipides est très intéressante pour éviter le stockage des graisses et favoriser l'élimination de celles qui sont stockées. On a également observé que cette augmentation de la thermogenèse par les capsaicinoïdes contenus dans le Piment s'accompagne d'une réduction de l'appétit.

En outre, le Piment est traditionnellement utilisé pour traiter les troubles circulatoires, en particulier l'hypercholestérolémie, ou certains troubles digestifs tels que les flatulences, les ulcères gastriques, les reflux gastro-oesophagiens et les infections intestinales.

Toutefois, le Piment présente souvent l'inconvénient d'irriter la muqueuse gastrique.

L'étude de YEOH K.G. et al. « How does chilli cause upper gastro-intestinal symptomes » J. Clin. Gastroenterol., 1995, 21, p. 87-90 démontre que les douleurs digestives induites par l'ingestion de 5 g de Piment contenant 0.048 % en poids de capsaicinoïdes seraient liées à la stimulation de terminaison nerveuse locale au niveau de l'oesophage.

Les populations en Europe et aux Etats-Unis apparaissent encore plus sensibles au caractère irritant de cet épice sur la muqueuse gastrique que les populations d'Asie car la consommation de Piment y est beaucoup plus faible.

Le problème d'irritation de la muqueuse gastrique due au Piment limite ainsi l'administration de capsaicinoïdes par voie orale.

Pour diminuer la sensation de brûlure que pourrait occasionner le piment après ingestion, le brevet US 5,273,754 propose d'associer à celui-ci une substance carminative rafraîchissante telle que la menthe poivrée, le menthol, la menthe verte, ou la carvone. Toutefois, ces substances sont fortement odorantes et peuvent incommoder les personnes qui n'apprécient pas le goût de menthe. Il peut être également souhaitable de disposer d'une composition à base de capsaicinoïdes sans arôme ni odeur.

US 6 022 718 concerne des compositions stimulant de thermogenèse, à base de capsaicinoides et une huile végétale.

La teneur en capsaicinoïdes du Piment frais varie de 0.01 à 510 µg/g selon REILLY.CA. (2001). L'oléorésine (ou résine de capsicum) est un extrait organique du Piment largement utilisé dans l'industrie alimentaire pour l'aromatisation de plats industriels. Il permet d'apporter un goût épicé de façon reproductible et sous forme concentrée.

L'oléorésine étant lipophile, les huiles acceptables pour une administration orale sont bien adaptées pour la formulation des capsaicinoïdes.

De manière tout à fait surprenante, les inventeurs ont découvert qu'en incorporant à cette huile un additif lipophile solide ou pâteux à température ambiante, la composition ainsi obtenue était dépourvue de tout effet irritant sur la muqueuse gastrique.

La présente invention a pour objet une composition pour une administration par voie orale contenant des capsaicinoïdes en association avec une base de formulation acceptable pour l'administration par voie orale, ladite base de formulation comprenant une huile et un additif lipophile solide ou pâteux à température ambiante.

On entend par base de formulation, une substance de support inerte dans laquelle sont introduits des éléments actifs.

La composition objet de la présente invention se présente avantageusement sous forme solide ou pâteuse à température ambiante.

L'additif lipophile représente avantageusement 5 à 20% en poids, de préférence 8 à 15% en poids de la composition.

Avantageusement, l'additif lipophile a un point de fusion compris entre 30 et 80°C si bien qu'il est solide ou pâteux à température ambiante et peut être fondu dans l'huile avec des équipement industriels standards.

L'additif lipophile est de préférence choisi parmi les cires, les mono-, di- ou triglycérides d'acides gras, les acides gras et les polyéthylènes glycol et les esters d'acide gras de polyéthylène glycol, ainsi que leurs mélanges.

Les cires peuvent être de la cire, d'abeille, de la cire candelilla, de la cire de carnauba, de la cire de polyéthylène oxydée ou de la cire de pétrole (ou cire microcristalline). On utilise avantageusement de la cire d'abeille.

Les mono-, di-, ou triglycérides d'acides gras peuvent être de différents degrés d'estérification. On utilise avantageusement le palmitostéarate de glycérol.

Les acides gras peuvent être choisis parmi l'acide palmitique, l'acide stéarique ou l'acide behenique, ainsi que leurs sels de calcium, sodium, potassium ou de magnésium.

Les polyéthylèneglycols et les esters d'acides gras du polyéthylèneglycol ont avantageusement un poids moléculaire compris entre 600 à 6000.

De manière préférée, l'additif lipophile est constitué d'un mélange de cire d'abeille et de palmitostéarate de glycérol, dont le ratio massique préféré est d'environ 1.

Le Piment est le fruit d'une plante herbacée annuelle de la famille des Solanacées. Les deux espèces les plus utilisées sont *Capsicum annuum* et *Capsicum frutescens* qui font l'objet de cultures notamment en Europe, en Afrique et en Amérique du nord et du Sud.

Outre les capsaicinoïdes, les fruits du Piment contiennent des caroténoides tels que le béta-carotène, la zeaxanthine, la violaxanthine, la capsanthine et la capsorubine (ces 2 derniers sont spécifiques du genre Capsicum).

Les capsaicinoïdes peuvent être incorporés à la composition sous la forme de poudre du fruit séché ou de résine de capsicum (ou oléorésine).

La résine de capsicum peut contenir entre 3 et 50 % en poids, de préférence entre 5 et 20 % en poids de capsaicinoïdes.

Les capsaicinoïdes sont avantageusement incorporés à la composition sous la forme de résine de capsicum.

Les capsaicinoïdes représentent avantageusement entre 0,02 et 5% en poids, de préférence 0,1 à 2 % en poids de la composition.

L'huile est avantageusement choisie parmi les huiles végétales telles que l'huile de soja, l'huile de tournesol, l'huile de maïs, l'huile d'olive ou l'huile de noix et parmi les huiles minérales telles que l'huile de pàraffine, ainsi que leurs mélanges.

La composition comprend avantageusement un ou plusieurs composants physiologiquement actifs autres que les capsaicinoïdes.

Ces autres composants physiologiquement actifs représentent avantageusement 10 à 30 % en poids de la composition et peuvent être choisis parmi des stimulateurs de la thermogenèse ou du transit.

Les travaux scientifiques réalisés depuis plusieurs années ont permis de démontrer que certains aliments ont la capacité de stimuler la thermogenèse : c'est notamment le cas de la caféine contenue dans le café et le thé. Par ailleurs, l'Ascophyllum nodosum est un stimulateur de transit.

Avantageusement, au moins un parmi les autres composants physiologiquement actifs est un végétal ou un extrait de celui-ci, choisi parmi le thé vert, l'algue Ascophyllum nodosum, le maté, le guarana, l'éphédra ou le citrus aurantium, ainsi que leurs mélanges, et/ou une huile de tournesol riche en acide linoléïque conjugué.

La composition selon la présente invention peut se présenter sous la forme d'une capsule molle ou dure. L'enveloppe des capsules molles ou dures est avantageusement en gélatine bovine, en gélatine de poisson, en hydroxypropylméthylcellulose ou en un autre polymère d'origine végétale ou animale.

Les capsules dures n'ayant pas un système de fermeture étanche, il est simplement nécessaire de mettre en oeuvre un procédé permettant de les étanchéifier (banderolage ou scellage interne).

La composition objet de la présente invention est préparée selon des techniques classiques connues par l'homme du métier :
**1)** L'additif lipophile est incorporé à l'huile qui est chauffée à une température suffisante pour permettre de faire fondre complètement l'additif lipophile et obtenir un mélange homogène,
**2)** Après refroidissement à environ 50 °C, les autres composants tels que l'oléorésine de capsicum et composants stimulateurs de la thermogenèse sont incorporés à ce mélange sous agitation,
**3)** Le mélange ainsi obtenu est refroidi à une température comprise entre 25 et 40 °C,
**4)** On procède éventuellement au remplissage de capsules molles ou dures par ce mélange.

Telle que formulée, la composition objet de la présente invention ne présente avantageusement aucune sédimentation des autres composants stimulateurs de la thermogenèse tels que l'extrait de thé vert ou bien stimulateur du transit tel que la poudre d'algue Ascophyllum.

La présente invention a également pour objet l'utilisation de la composition comme aliment, complément alimentaire ou produit diététique (aliment destiné à une alimentation particulière).

La composition peut être notamment incorporée dans des aliments qui sont préparés industriellement ou artisanalement, tels que des huiles, du beurre, de la margarine, des pâtes à tartiner du chocolat. Elle peut aussi se présenter sous la forme d'une poudre à diluer dans l'eau ou de barres alimentaires.

La composition selon la présente invention est avantageusement utilisée pour stimuler la thermogenèse, éventuellement avec réduction d'appétit, sans qu'elle produise d'effet irritant sur la muqueuse gastrique.

De manière avantageuse, la composition selon la présente invention peut être ainsi utilisée pour réduire ou prévenir l'apparition de la cellulite, ou pour réduire ou prévenir la surcharge pondérale.

De manière très avantageuse, la composition selon la présente invention peut être utilisée comme complément alimentaire destiné aux personnes qui souhaitent réduire leur masse grasse tout en réduisant le stockage des graisses alimentaires et en éliminant plus de graisses.

La présente invention a également pour objet l'utilisation de la composition comme médicament.

Elle est avantageusement utilisée pour stimuler la thermogenèse, éventuellement avec réduction d'appétit, sans qu'elle produise d'effet irritant sur la muqueuse gastrique.

Elle permet ainsi de traiter ou prévenir l'obésité, les troubles circulatoires ou certains troubles digestifs tels que les flatulences, les ulcères gastriques, les reflux gastro-oesophagiens et les infections intestinales.

Elle peut également être utilisée pour le traitement symptomatique du zona, des douleurs rhumatismales, des neuropathies diabétiques et du psoriasis.

### A) Exemples de compositions sous forme de capsules molles

Des compositions ont été préparées sous forme de capsule molle à partir d'oléorésine de capsicum, d'huile de soja et d'environ 5 % en poids de cire d'abeille et environ 5 % poids de palmitostéarate de glycérol.

### Formulation 1 :

- 15 mg d'oléorésine de capsicum,
- 150 mg d'extrait de thé vert,
- 75 mg d'huile de tournesol riche en acide linoléïque conjugué,
- 200 mg d'huile de soja,
- 16 mg de lécithine de soja,
- 29 mg de cire jaune d'abeille,
- 25 mg de palmitostéarate de glycérol,
Enveloppe de la capsule molle : Gélatine, Glycérol, Sorbitol, colorants.

### Formulation 2 :

- 15 mg d'oléorésine de capsicum,
- 150 mg de poudre d'Ascophyllum nodosum,
- 75 mg d'huile de tournesol riche en acide linoléïque conjugué,
- 200 mg d'huile de soja,
- 16 mg de lécithine de soja,
- 29 mg de cire jaune d'abeille,
- 25 mg de palmitostéarate de glycérol,
Enveloppe de la capsule molle : Gélatine, Glycérol, Sorbitol, colorants.

Les formulations 1 et 2 ont été préparées de la façon suivante :
**1)** Dans l'huile de soja chauffée à 60 °C environ, faire fondre la cire d'abeille et le palmitostéarate de glycérol,
**2)** Refroidir le mélange ci-dessus à 50 °C environ et incorporer sous agitation l'huile de tournesol riche en CLA, la lécithine de soja, l'oléorésine de capsicum et l'extrait de thé vert ou l'Ascophyllum nodosum,
**3)** Refroidir le mélange à une température comprise entre 25 et 40 °C,
**4)** Procéder au remplissage des capsules molles selon le procédé classique.
Les formulations 1 et 2 se présentent sous forme pâteuse à température ambiante.

Dans une étude réalisée chez la femme, YOSHIOKA M. et al. « Effects of red pepper added to high fat and high carbohydrate meals on energy metabolism » Br. J. Nut., 1998, n° 80, p. 503-510, les auteurs émettent l'hypothèse d'une différence de réponse métabolique suite à la prise de Capsicum entre les hommes et les femmes.

En effet, les muscles des femmes contiennent un pourcentage plus élevé de fibres de type 1 que les hommes. Or, ces fibres de type 1 renferment 3 fois plus de récepteurs béta-adrénergiques que les fibres de type 2. Cette différence expliquerait que dans cette étude, la réponse métabolique chez les femmes conduise à une oxydation beaucoup plus importante des lipides que chez les hommes.

La formulation 1 contient un extrait de thé vert riche en caféine qui est un stimulant du système béta-adrénergique. Pour cette raison, la formulation 1 sera avantageusement utilisée par l'homme.

### B) Tests in vitro de libération du contenu de la capsule molle

Ces tests ont été réalisés à partir de la formulation 1 à l'aide d'un appareil de mesure du temps de dissolution, (appareil à palette tournante) décrit dans la pharmacopée européenne.

Les capsules molles sont placées dans 1000 ml d'eau à 37°C.

Les résultats suivants ont été obtenus :

| Cire d'abeille (% en poids par rapport à la composition totale) | Palmitostéarate de glycérol (% en poids par rapport à la composition totale) | Remplissage des capsules molles | Temps de dissolution |
|---|---|---|---|
| 2% | 0% | OK | Libération complète en moins de 30 min |
| 3 % | 0 % | OK | |
| 4 % | 0 % | OK | |
| 5 % | 0 % | OK | |
| 6 % | 0% | Viscosité trop importante pour le remplissage des capsules molles | - |
| 5 % | 3 % | OK | Libération complète en 90 min |
| 5 % | 6 % | Viscosité trop importante pour le remplissage des capsules molles | - |
| 5% | 5 % | OK | Libération complète en 120 min |

Ces résultats montrent que les temps de dissolution in vitro des compositions dépend de la proportion de cire d'abeille et de palmitostéarate de glycérol incorporée.

Certaines proportions entraînent une élévation du point de fusion du mélange. En conséquence, la température de remplissage du mélange huileux dans la capsule molle devient trop élevée pour permettre le remplissage des capsules molles.

Une dissolution complète en environ 120 min est obtenue avec une composition contenant 5% en poids de cire d'abeille et 5% en poids de palmitostéarate de glycérol.

Le temps de dissolution minimum souhaitable est d'au moins 1 heure. II est de préférence d'au moins 90 minutes, et de manière encore plus préférée d'au moins 120 minutes.

### C) Test de tolérance digestive sur des sujets sains

L'amélioration de la tolérance digestive du capsicum par l'association cire d'abeille-palmitostéarate de glycérol a été testée sur 20 sujets sains prenant successivement et dans un ordre aléatoire :
- une composition sans cire et sans palmitostéarate de glycérol,
- pour les femmes : la composition 2, et pour les hommes : la composition 1.

| | Pourcentage des sujets présentant des problèmes de tolérance digestive tels que des douleurs gastriques et des sensations de brûlures | Pourcentage des sujets présentant une bonne tolérance digestive |
|---|---|---|
| Composition sans cire et sans palmitostéarate de glycérol | 55 % | 45 % |
| Composition contenant 5% en poids de cire d'abeille et 5% en poids de palmitostéarate de glycérol | 0 % | 100 % |

Ce test montre que les compositions renfermant un mélange de cire d'abeille et de palmitostéarate de glycérol en tant qu'additif lipophile solide ou pâteux à température ambiante, entraînent pour une même dose d'oléorésine de capsicum moins de brûlures digestives que les capsules molles qui ne contiennent aucun additif lipophile solide ou pâteux à température ambiante.

## Revendications

1. Composition stimulant la thermogenèse, pour une administration par voie orale, contenant des capsaicinoïdes en association avec une base de formulation acceptable pour l'administration par voie orale, **caractérisée en ce que** ladite base de formulation comprend une huile végétale et/ou minérale et un additif lipophile solide ou pâteux à température ambiante, l'additif lipophile solide ou pâteux à température ambiante représentant 5 à 20 % en poids, de préférence 8 à 15 % en poids de la composition.

2. Composition selon la revendication 1, **caractérisée en ce que** l'additif lipophile est choisi parmi les cires, les mono-, di- ou triglycérides d'acides gras, les acides gras et les polyéthylènes glycol et les esters d'acide gras de polyéthylène glycol, ainsi que leurs mélanges.

3. Composition selon l'une quelconque des revendications 1 à 2, **caractérisée en ce que** l'additif lipophile est un mélange de cire d'abeille et de palmitostéarate de glycérol.

4. Composition selon la revendication 3, **caractérisée en ce que** le ratio massique entre la cire d'abeille et le palmitostéarate de glycérol est d'environ 1.

5. Composition selon l'une quelconque des revendications 1 à 4, **caractérisée en ce que** les capsaicinoïdes sont présents dans la composition sous la forme de résine de capsicum.

6. Composition selon l'une quelconque des revendications 1 à 5, **caractérisée en ce que** les capsaicinoïdes représentent 0,02 à 5 % en poids, de préférence 0,1 à 2 % en poids de la composition.

7. Composition selon l'une quelconque des revendications 1 à 6, **caractérisée en ce que** l'huile végétale est choisie parmi l'huile de soja, l'huile de tournesol, l'huile de maïs, l'huile d'olive ou l'huile de noix et l'huile minérale est une huile de paraffine.

8. Composition selon l'une quelconque des revendications 1 à 7, **caractérisée en ce qu'**elle comprend en outre un ou plusieurs composants physiologiquement actifs autres que les capsaicinoïdes.

9. Composition selon la revendication 8, **caractérisée en ce que** le ou les autres composants physiologiquement actifs représentent 10 à 30 % en poids de la composition.

10. Composition selon la revendication 8 ou 9, **caractérisée en ce que** au moins un parmi les autres composants physiologiquement actifs est un végétal ou un extrait de celui-ci, choisi parmi le thé vert, l'algue Ascophyllum nodosum, le maté, le guarana, l'éphédra ou le citrus aurantium, ainsi que leurs mélanges.

11. Composition selon l'une quelconque des revendications 8 à 10, **caractérisée en ce que** au moins un parmi les autres composants physiologiquement actifs est une huile de tournesol riche en acide linoléïque conjugué.

12. Composition selon l'une quelconque des revendications 1 à 11, **caractérisée en ce qu'**elle se présente sous forme solide ou pâteuse à température ambiante.

13. Composition selon l'une quelconque des revendications 1 à 12, **caractérisée en ce qu'**elle est sous forme d'une capsule molle ou dure.

## Claims

1. Composition which stimulates thermogenesis, for oral administration, comprising capsaicinoids in combination with a formulation base which is acceptable for oral administration, **characterized in that** said formulation base comprises a vegetable and/or mineral oil and a lipophilic additive which is solid or pasty at room temperature, the lipophilic additive which is solid or pasty at room temperature amounting to 5 to 20% by weight, preferably 8 to 15% by weight, of the composition.

2. Composition according to Claim 1, **characterized in that** the lipophilic additive is selected from among waxes, fatty acid mono-, di- or triglycerides, fatty acids and polyethylene glycols and the fatty acid polyethylene glycol esters, as well as their mixtures.

3. Composition according to either one of Claims 1 and 2, **characterized in that** the lipophilic additive is a mixture of beeswax and glycerol palmitostearate.

4. Composition according to Claim 3, **characterized in that** the weight ratio between the beeswax and the glycerol palmitostearate is approximately 1.

5. Composition according to any one of Claims 1 to 4, **characterized in that** the capsaicinoids are present in the composition in the form of capsicum resin.

6. Composition according to any one of Claims 1 to 5, **characterized in that** the capsaicinoids represent 0.02 to 5% by weight, preferably 0.1 to 2% by weight, of the composition.

7. Composition according to any one of Claims 1 to 6, **characterized in that** the vegetable oil is selected from among soya oil, sunflower oil, corn oil, olive oil or nut oil and the mineral oil is a liquid paraffin.

8. Composition according to any one of Claims 1 to 7, **characterized in that** it comprises furthermore one or more physiologically active components other than the capsaicinoids.

9. Composition according to Claim 8, **characterized in that** the other physiologically active component(s) amount to 10 to 30% by weight of the composition.

10. Composition according to Claim 8 or 9, **characterized in that** at least one among the other physiologically active components is a plant or a plant extract selected from among green tea, the alga Ascophyllum nodosum, mate tea, guarana, ephedra or citrus aurantium, as well as their mixtures.

11. Composition according to any one of Claims 8 to 10, **characterized in that** at least one among the other physiologically active components is a sunflower oil which is high in conjugated linoleic acid.

12. Composition according to any one of Claims 1 to 11, **characterized in that** it is presented in a form which is solid or pasty at room temperature.

13. Composition according to any one of Claims 1 to 12, **characterized in that** it is in the form of soft or hard capsules.

## Patentansprüche

1. Zusammensetzung, welche die Wärmebildung stimuliert, für eine Verabreichung auf oralem Wege, welche Capsaicinoide in Verbindung mit einer Formulierungsgrundlage, welche für eine Verabreichung auf oralem Wege annehmbar ist, enthält, **dadurch gekennzeichnet, dass** die Formulierungsgrundlage ein pflanzliches und/oder mineralisches Öl und einen bei Umgebungstemperatur festen oder pastenartigen lipophilen Zusatzstoff umfasst, wobei der bei Umgebungstemperatur feste oder pastenartige lipophile Zusatzstoff 5 bis 20 Gew.-%, vorzugsweise 8 bis 15 Gew.-% der Zusammensetzung ausmacht.

2. Zusammensetzung nach Anspruch 1, **dadurch gekennzeichnet, dass** der lipophile Zusatzstoff unter den Wachsen, den Mono-, Di- oder Triglyceriden von Fettsäuren, den Fettsäuren und den Polyethylenglycolen und den Fettsäureestern von Polyethylenglycol wie auch deren Mischungen ausgewählt wird.

3. Zuammensetzung nach einem der Ansprüche 1 bis 2, **dadurch gekennzeichnet, dass** der lipophile Zusatzstoff eine Mischung von Bienenwachs und Glycerolpalmitostearat ist.

4. Zusammensetzung nach Anspruch 3, **dadurch gekennzeichnet, dass** das Massenverhältnis zwischen dem Bienenwachs und dem Glycerolpalmitostearat ungefähr 1 beträgt.

5. Zusammensetzung nach einem der Ansprüche 1 bis 4, **dadurch gekennzeichnet, dass** die Capsaicinoide in der Zusammensetzung in Form von Capsicum-Harz vorliegen.

6. Zusammensetzung nach einem der Ansprüche 1 bis 5, **dadurch gekennzeichnet, dass** die Capsaicinoide 0,02 bis 5 Gew.-%, vorzugsweise 0,1 bis 2 Gew.-% der Zusammensetzung ausmachen.

7. Zusammensetzung nach einem der Ansprüche 1 bis 6, **dadurch gekennzeichnet, dass** das pflanzliche Öl aus Sojaöl, Sonnenblumenöl, Maisöl, Olivenöl oder Walnussöl ausgewählt wird und das mineralische Öl ein Paraffinöl ist.

8. Zusammensetzung nach einem der Ansprüche 1 bis 7, **dadurch gekennzeichnet, dass** sie außerdem einen oder mehrere andere physiologisch wirksame Bestandteile als die Capsaicinoide umfasst.

9. Zusammensetzung nach Anspruch 8, **dadurch gekennzeichnet, dass** der oder die anderen physiologisch wirksamen Bestandteile 10 bis 30 Gew-.% der Zusammensetzung ausmachen.

10. Zusammensetzung nach Anspruch 8 oder 9, **dadurch gekennzeichnet, dass** wenigstens einer unter den anderen physiologisch wirksamen Bestandteilen ein Pflanzenmaterial oder ein Extrakt von jenem, ausgewählt unter grünem Tee, der Alge Ascophyllum nodosum, Mate, Guarana, Ephedra oder Citrus aurantium wie auch deren Mischungen, ist.

11. Zusammensetzung nach einem der Ansprüche 8 bis 10, **dadurch gekennzeichnet, dass** wenigstens einer unter den anderen physiologisch wirksamen Bestandteilen ein an konjugierter Linolsäure reiches Sonnenblumenöl ist.

12. Zusammensetzung nach einem der Ansprüche 1 bis 11, **dadurch gekennzeichnet, dass** sie in bei Umgebungstemperatur fester oder pastenartiger Form vorliegt.

13. Zusammensetzung nach einem der Ansprüche 1 bis 12, **dadurch gekennzeichnet, dass** sie in Form einer weichen oder harten Kapsel vorliegt.
